# EUROPEAN PATENT APPLICATION

(11) **EP 4 678 159 A1**
(43) Date of publication of application: **14.01.2026**
(21) Application number: 24803458.9
(22) Date of filing: 07.05.2024
(51) Int. Cl.: A61K 8/31, A61K 8/81, A61Q 1/00, A61Q 19/00

(54) **OIL AGENT FOR COSMETIC PREPARATIONS**

(30) Priority: 11.05.2023 JP 2023078941
(71) Applicant: NOF Corporation, Shibuya-ku Tokyo 150-6019 (JP)
(72) Inventor: SAKATSUME Yuma, Amagasaki-shi, Hyogo 660-0095 (JP); MORIKAWA Toshiyuki, Amagasaki-shi, Hyogo 660-0095 (JP)
(74) Representative: Williams Powell
(86) International application number: PCT/JP2024/016921
(87) International publication number: WO 2024/232347

(57) **Abstract**

An oil agent for cosmetic preparations, including, component (A): from 5 to 60 mass% of an isoparaffin having an average carbon number from 50 to 250, component (B): from 30 to 90 mass% of an isoparaffin having an average carbon number from 20 to 24, and component (C): from 0.3 to 10 mass% of an isoparaffin or a paraffin having 14 carbon atoms, in which the mass ratio of component (A) to component (C) ((A)/(C)) is from 1 to 150, and the mass ratio of component (B) to component (C) ((B)/(C)) is from 5 to 100.

## Description

### Technical field

The present invention relates to a liquid oil agent for cosmetic preparations.

### Background Art

Oil agents for cosmetic preparations can be broadly divided into three categories: solid oil agents, semi-solid oil agents, and liquid oil agents. Among them, liquid oil agents are used to maintain the moisture content of the stratum corneum and to improve compatibility with dirt and the like to remove them. Examples of liquid oil agents include highly polar ester oils synthesized from an alcohol and a carboxylic acid, low polar hydrocarbon oils composed only of carbon atoms and hydrogen atoms, and silicone oils containing silicon atoms, such as dimethicone. Although one of these oil agents may be used alone for various purposes, two or more of these oil agents may also be used in combination. In such a case, an oil agent should have compatibility with another oil agent as its function.

For example, PTL 1 and PTL 2 disclose cosmetic preparations containing, as an oil agent, an ester containing a branched alkyl chain. The esters contained in these cosmetic preparations have excellent compatibility with silicone oils which are used to improve the feeling of use and long-wear property of the cosmetic.

Furthermore, among hydrocarbon oils such as isoparaffin, hydrocarbon oils having an average carbon number of 24 or less are known to have excellent compatibility with silicone oils. However, hydrocarbon oils having an average carbon number of 50 or more may have poor compatibility with silicone oils. One possible solution to this problem is to combine a hydrocarbon oil with excellent compatibility with a hydrocarbon oil with poor compatibility, and thereby mix the hydrocarbon oil with poor compatibility with a silicone oil.

One of the effects of combining two types of hydrocarbon oils in this manner is reduction of the viscosity of the hydrocarbon oil having an average carbon number of 50 or more. For example, PTL 3 discloses a cosmetic composition containing a paraffin mixture containing an isoparaffin having from 12 to 16 carbon atoms and having a boiling point ranging from 185 to 215°C, and a heavy liquid isoparaffin having a kinematic viscosity at 210°F (98.9°C) of from 200 to 10,000 mm²/s. Furthermore, PTL 4 discloses a liquid skin cosmetic preparation including a combination of a heavy liquid isoparaffin and any one of a liquid paraffin, a liquid isoparaffin, and squalane. These compositions include a combination of a heavy liquid isoparaffin, which is highly viscous in itself, and a hydrocarbon oil having a low viscosity to improve ease of application to hair and ease of spreading on skin.

However, although these compositions are excellent in reducing viscosity, such a low viscosity may cause dripping of the composition before spreading it upon application to hair or skin. Furthermore, these compositions have high wetting properties, and thus may not provide the feeling of thickness upon application to hair or skin and may be poor in the feeling of spreading the composition.

Thus, there is a demand for an oil agent for cosmetic preparations that is highly compatible with silicone oils, has excellent spreadability, has a viscosity that enables suppression of dripping, and exhibits wetting properties that enables provision of the feeling of thickness upon application.

### Citation List

### Patent Literature

PTL 1: JP 2006-290787 A
PTL 2: JP H10-265324 A
PTL 3: JP 2014-221722 A
PTL 4: JP 2018-188392 A

### Summary of Invention

### Technical Problem

The present invention has been made to solve the above-mentioned problems, and the object thereof is to provide an oil agent for cosmetic preparations that is highly compatible with silicone oils, has excellent spreadability, has a viscosity that enables suppression of dripping, and exhibits wetting properties that enables provision of the feeling of thickness upon application.

### Solution to Problem

As a result of intensive studies by the inventors to solve the above problems, the inventors found that the above problems can be solved by combining an isoparaffin having a specific average carbon number, and an isoparaffin or a paraffin having a specific number of carbon atoms in a specific mass ratio, and thus made the present invention.

The present invention is the following oil agent for cosmetic preparations.

An oil agent for cosmetic preparations, including,
component (A): from 5 to 60 mass% of an isoparaffin having an average carbon number from 50 to 250,
component (B): from 30 to 90 mass% of an isoparaffin having an average carbon number from 20 to 24, and
component (C): from 0.3 to 10 mass% of an isoparaffin or a paraffin having 14 carbon atoms, in which
the mass ratio of component (A) to component (C) ((A)/(C)) is from 1 to 150, and the mass ratio of component (B) to component (C) ((B)/(C)) is from 5 to 100.

### Advantageous Effects of Invention

The oil agent for cosmetic preparations of the present invention is highly compatible with silicone oils, has excellent spreadability, has a viscosity that enables suppression of dripping, and exhibits wetting properties that enables provision of the feeling of thickness upon application.

### Description of Embodiments

Hereinafter, embodiments of the present invention will be described. However, the present invention is not limited to the embodiments described herein, and various modifications are possible without departing from the spirit and scope of the present invention.

Note that, as used herein, any numerical range defined using "from ... to" is intended to include numerical values before and after "to" (the upper and lower limits). For example, "from 2 to 5" means 2 or more and 5 or less.

Furthermore, regarding any numerical range described herein, the upper or lower limit value of the numerical range can be replaced with a value presented in an example or a value that is uniquely derived from an example.

It is understood that values described herein are subject to the inherent variation characteristic of a measurement technique used to determine the values. In addition, numerical values should be interpreted in light of the number of significant digits and by applying rounding techniques.

### <Oil agent for Cosmetic Preparations>

An oil agent for cosmetic preparations of the present invention contains component (A), component (B), and component (C), and the total content of components (A), (B), and (C) is 100 mass%. In this specification, when a composition contains multiple substances corresponding to a component, the content of that component in the composition means the total content of the multiple substances contained in the composition, unless otherwise noted.

Each component will be described below.

### [Component (A)]

Component (A) used in the present invention is an isoparaffin having an average carbon number from 50 to 250, in other words, a mixture of long-chain hydrocarbons having a side chain, and is typically obtained by hydrogenating a polymer made from isobutene and n-butene. Such components include those generally classified as "heavy liquid isoparaffins" in the cosmetics field.

The average carbon number of the isoparaffin is from 50 to 250, preferably from 60 to 230, more preferably from 70 to 210, and even more preferably from 80 to 190. An excessively small average carbon number causes decrease in viscosity, which may cause dripping and lessen the feeling of the thickness upon application. On the other hand, an excessively large average carbon number causes reduction of compatibility with a silicone oil and increase in viscosity, which may reduce spreadability on hair or skin.

The average carbon number in this specification is a value derived from peak area measurements and results of mass analysis obtained by using a gas chromatography-mass spectrometry (GC-MS) measuring device (JMS-T2000GC AccuTOF (registered trademark) GC-Alpha, manufactured by JEOL Ltd.).

Specific examples of component (A) include "PARLEAM 18" (having an average carbon number of 72), "PARLEAM 24" (having an average carbon number of 96), and "PARLEAM 46" (having an average carbon number of 184), all manufactured by NOF CORPORATION. As component (A), one or more of these isoparaffins having different average carbon numbers can be used.

### [Component (B)]

Component (B) used in the present invention is an isoparaffin having an average carbon number from 20 to 24, is a mixture of medium-chain hydrocarbons having a side chain, and is typically obtained by hydrogenating a polymer made from isobutene and n-butene.

An excessively small average carbon number causes decrease in viscosity, which may cause dripping and lessen the feeling of the thickness upon application. On the other hand, an excessively large average carbon number causes reduction of compatibility with a silicone oil and increase in viscosity, which may reduce spreadability on hair or skin.

Component (B) can be synthesized, for example, using the following method. First, a mixed gas of isobutene and n-butene can be processed using a known method, for example, cationic polymerization using a catalyst, to obtain a polymer. Then, the resulting polymer can be hydrogenated to obtain a hydrogenated polymer, which can be subjected to purification such as adsorption treatment and distillation to obtain component (B). As component (B), one or more of these isoparaffins having different average carbon numbers can be used.

### [Component (C)]

Component (C) used in the present invention is an isoparaffin or a paraffin having 14 carbon atoms, specifically isotetradecane or tetradecane. Component (C) is preferably isotetradecane.

Specific examples of component (C) include "PARAFOL 14-97" manufactured by Sasol Ltd. As component (C), isotetradecane and tetradecane may be used in combination.

### [Content of Each Component]

The lower limit of the content of component (A) is 5 mass%, preferably 10 mass%, more preferably 15 mass%, and even more preferably 20 mass%. An excessively low content of component (A) causes decrease in viscosity, which may cause dripping and lessen the feeling of the thickness upon application. The upper limit of the content of component (A) is 60 mass%, preferably 55 mass%, more preferably 50 mass%, and even more preferably 40 mass%. An excessively high content of component (A) causes reduction of compatibility with a silicone oil and increase in viscosity, which may reduce spreadability on hair or skin. The content of component (A) is, for example, in the range of from 5 to 60 mass%, preferably from 10 to 55 mass%, more preferably from 15 to 50 mass%, and even more preferably from 20 to 40 mass%.

The lower limit of the content of component (B) is 30 mass%, preferably 40 mass%, more preferably 45 mass%, and even more preferably 50 mass%. An excessively low content of component (B) causes reduction of compatibility with a silicone oil and increase in viscosity, which may reduce spreadability on hair or skin. The upper limit of the content of component (B) is 90 mass%, preferably 80 mass%, more preferably 75 mass%, and even more preferably 70 mass%. An excessively high content of component (B) causes decrease in viscosity, which may cause dripping and lessen the feeling of the thickness upon application. The content of component (B) is, for example, in the range of from 30 to 90 mass%, preferably from 40 to 80 mass%, more preferably from 45 to 75 mass%, and even more preferably from 50 to 70 mass%.

The lower limit of the content of component (C) is 0.3 mass%, preferably 0.5 mass%, more preferably 1 mass%, and even more preferably 3 mass%. An excessively low content of component (C) causes reduction of compatibility with a silicone oil and increase in viscosity, which may reduce spreadability on hair or skin. The upper limit of the content of component (C) is 10 mass%, preferably 9 mass%, more preferably 8 mass%, and even more preferably 7 mass%. An excessively high content of component (C) causes decrease in viscosity, which may cause dripping and lessen the feeling of the thickness upon application. The content of component (C) is, for example, in the range of from 0.3 to 10 mass%, preferably from 0.5 to 9 mass%, more preferably from 1 to 8 mass%, and even more preferably from 3 to 7 mass%.

In the present invention, the mass ratio of component (A) to component (C) ((A)/(C)) is from 1 to 150, preferably from 2 to 100, more preferably from 3 to 70, and even more preferably from 4 to 40. An excessively low mass ratio ((A)/(C)) causes decrease in viscosity, which may cause dripping and lessen the feeling of the thickness upon application. An excessively high mass ratio ((A)/(C)) causes reduction of compatibility with a silicone oil and increase in viscosity, which may reduce spreadability on hair or skin.

In the present invention, the mass ratio of component (B) to component (C) ((B)/(C)) is from 5 to 100, preferably from 8 to 85, more preferably from 9 to 70, and even more preferably from 10 to 60. An excessively low mass ratio ((B)/(C)) causes decrease in viscosity, which may cause dripping and lessen the feeling of the thickness upon application. An excessively high mass ratio ((B)/(C)) causes reduction of compatibility with a silicone oil and increase in viscosity, which may reduce spreadability on hair or skin.

The oil agent for cosmetic preparations of the present invention can be produced by mixing components (A) to (C) by using a known mixing method and a known mixing device, such as a mixer (e.g., a mill) or a kneader. The mixture may be heated upon mixing, and if heated, the mixture is preferably heated to 120°C or less.

The viscosity at 25°C of the oil agent for cosmetic preparations of the present invention is preferably 100 mPa·s or more and less than 5,000 mPa·s, and more preferably 200 mPa·s or more and less than 1,500 mPa·s. The viscosity within the above ranges allows the oil agent for cosmetic preparations to be spread on hair or skin without dripping.

### [Other components]

The oil agent for cosmetic preparations of the present invention can be used in hair cosmetic preparations and skin cosmetic preparations, and may contain another component commonly used in cosmetic preparations or pharmaceuticals, as needed, as long as the other component does not impair the characteristics of the present invention.

### Examples

The present invention has been described above by presenting preferred embodiments for ease of understanding. Hereinafter, the present invention will be described in more detail and specifically with reference to synthesis examples, comparative synthesis examples, examples, and comparative examples. However, the above description and the following synthesis examples and the like are provided for illustrative purposes only and are not provided for the purpose of limiting the present invention.

### [Examples 1 to 9, Comparative Examples 1 to 7]

### <Preparation of Oil Agent for Cosmetic Preparations>

Oil agents for cosmetic preparations of Examples 1 to 9 and Comparative Examples 1 to 7 were prepared by sequentially adding and uniformly mixing components (A), (B), (B'), and (C) shown in Tables 1 and 2.

As the components shown in Tables 1 and 2, commercially available products for cosmetics and synthetic products were used. The product names of #1 to #3 in Tables 1 and 2 are as follows. Note that the content of each component shown in Tables 1 and 2 is in mass%.
#1: "PARLEAM 24" manufactured by NOF CORPORATION
#2: "PARLEAM 46" manufactured by NOF CORPORATION
#3: "PARLEAM 18" manufactured by NOF CORPORATION

### (Synthesis Example 1: Hydrogenated Polyisobutene with Average Carbon Number of 24)

In an autoclave, 360 g of a mixed gas containing a mixed gas of olefins having 4 carbon atoms (which contains 30 mass% of isobutene and 43 mass% of n-butene) and 27 mass% of butane gas was placed, and polymerize the mixed gas in the presence of an aluminum chloride catalyst to obtain a polymer of isobutene and n-butene. The catalyst was removed by treatment with a caustic alkali aqueous solution and a water washing process. Next, the water-washed polymer was placed in a four-neck flask and heated in an oil bath. Unreacted gas components were removed by nitrogen bubbling at an internal temperature of 40°C, and then polymers having 16 or less carbon atoms were removed at an internal temperature of 140°C at a pressure reduction degree of 5 kPa. Then, the residue was hydrogenated in an autoclave in the presence of a hydrogenation catalyst (0.5% Pd/C alumina catalyst) at a hydrogen pressure of 3 MPa and 220°C to obtain 120 g of a hydrogenated polymer. The hydrogenated polymer was subjected to separation treatment by simple distillation to obtain hydrogenated polyisobutene having an average carbon number of 24.

### (Synthesis Example 2: Hydrogenated Polyisobutene with Average Carbon Number of 20)

As one of the fractions obtained from the simple distillation in Synthesis Example 1, hydrogenated polyisobutene having an average carbon number of 20 was obtained.

### (Comparative Synthesis Example 1: Hydrogenated Polyisobutene with Average Carbon Number of 36)

Polymerization and water washing were carried out in the same manner as in Synthesis Example 1, except that a mixed gas containing 20 mass% of isobutene, 30 mass% of n-butene, and 50 mass% of butane gas was used instead of the mixed gas in Synthesis Example 1. Subsequently, only the removal of unreacted gas components was carried out by nitrogen bubbling, and then hydrogenation was carried out to obtain hydrogenated polyisobutene having an average carbon number of 36.

### <Average Carbon number>

The average carbon number was determined from measurement results of the peak area and mass analysis obtained by using a gas chromatography-mass spectrometry (GC-MS) measuring device.
- Device: JMS-T2000GC AccuTOF (registered trademark) GC-Alpha, manufactured by JEOL Ltd.
- Conditions of GC analysis
   Column: DB-WAX (length: 30 m, inner diameter (diameter): 0.25 mm, film thickness: 0.25 µm)
   Conditions: from 50 to 250°C, programmed temperature analysis (3°C/min)
- Conditions of MS analysis
   Ionization method: EI
   Molecular weight range: m/z, 35 to 800

### <Evaluation of Oil agent for Cosmetic Preparations>

Each of the oil agents for cosmetic preparations of Examples 1 to 9 and Comparative Examples 1 to 7 was evaluated in terms of viscosity, compatibility, and thickness upon application as described in (1) to (3) below. The evaluation results are shown in Tables 1 and 2.

Note that panelists conducting the evaluations were trained in a preliminary evaluation test conducted prior to the evaluation tests such that the levels of evaluation corresponding to their respective scores were roughly the same, to equalize evaluation criteria of the panelists.

### (1) Viscosity

Each of the oil agents for cosmetic preparations of Examples and Comparative Examples was subjected to measurement by using a rheometer (MCR302, manufactured by Anton Paar Japan K.K.). Based on the measured viscosity (mPa·s, 25°C), the viscosity of the oil agent for cosmetic preparations was evaluated using a four grade scale from "A" to "D" according to the following evaluation criteria. "A" and "B" were considered to be acceptable.

### <Evaluation Criteria>

A: The viscosity is 200 mPa·s or more and less than 1,500 mPa·s
B: The viscosity is 100 mPa·s or more and less than 200 mPa·s, or is 1,500 mPa·s or more and less than 5,000 mPa·s
C: The viscosity is 50 mPa·s or more and less than 100 mPa·s, or is 5,000 mPa·s or more and less than 15,000 mPa·s
D: The viscosity is less than 50 mPa·s or 15,000 mPa·s or more

### (2) Compatibility

Into a container, 5 g of the oil agent for cosmetic preparations of each of Examples and Comparative Examples and 5 g of a silicone oil (dimethicone, KF-96A-20cs, manufactured by Shin-Etsu Chemical Co., Ltd.) were placed, and mixed with a stirrer at 25°C by using a 1.5 cm stir bar at a stirring rate such that the liquid surface was drawn in by a vortex at the center of the stirring. The state of mixing was visually observed, and the time until the mixture was uniformly mixed without haze due to concentration gradient was measured. Based on the measured time, the compatibility of the oil agent for cosmetic preparations was evaluated using a four grade scale from "A" to "D" according to the following evaluation criteria. "A" and "B" were considered to be acceptable.

### <Evaluation Criteria>

A: The time is less than 1 minute
B: The time is 1 minute or more and less than 5 minutes
C: The time is 5 minutes or more and less than 10 minutes
D: The time is more than 10 minutes or a uniform mixture could not be formed

### (3) Thickness upon Application

A test for evaluating feeling of use was conducted by 20 panelists. On the back of a hand, 0.5 g of the oil agent for cosmetic preparations of each of Examples and Comparative Examples was taken, and spread over the entire back of the hand using the index finger and middle finger of the other hand. The thickness upon application was evaluated according to the following absolute evaluation criteria. The total score of evaluation results of the panelists was calculated, and based on the total score, the thickness upon application was evaluated using a four grade scale from "A" to "D" according to the following evaluation criteria. "A" and "B" were considered to be acceptable.

### <Absolute Evaluation Criteria>

### (Score): (Evaluation)

3: The oil agent provided sufficient feeling of thickness.
2: The oil agent provided feeling of thickness.
1: The oil agent did not provide feeling of thickness.
0: The oil agent did not provide feeling of thickness, and the panelist felt that the oil agent ran out before he/she could spread it over the entire back of his/her hand.

### <Evaluation by Four Grade Scale Based on Total Score>

A: Total score from 50 to 60 points
B: Total score from 40 to 49 points
C: Total score from 30 to 39 points
D: Total score less than 30 points

**Table 1**

| | | | Examples | | | | | | | | | | | | | | | | | |
|---|---|---|---|---|---|---|---|---|---|---|---|---|---|---|---|---|---|---|---|---|
| | | | 1 | | 2 | | 3 | | 4 | | 5 | | 6 | | 7 | | 8 | | 9 | |
| Oil agent | Component (A) | Hydrogenated polyisobutene #1 with average carbon number of 96 | 30 | | 30 | | 15 | | 45 | | 50 | | 30 | | - | | - | | 30 | |
| | | Hydrogenated polyisobutene #2 with average carbon number of 184 | - | | - | | - | | - | | - | | - | | 30 | | - | | - | |
| | | Hydrogenated polyisobutene #3 with average carbon number of 72 | - | | - | | - | | - | | - | | - | | - | | 30 | | - | |
| | Component (B) | Synthesis Example 1 | 65 | | - | | 79 | | 48 | | 49 | | 69 | | - | | 65 | | 65 | |
| | | Synthesis Example 2 | - | | 64 | | - | | - | | - | | - | | 64 | | - | | - | |
| | Component (C) | Isotetradecane | 5 | | 6 | | 6 | | 7 | | 1 | | 1 | | 6 | | 5 | | - | |
| | | Tetradecane | - | | - | | - | | - | | - | | - | | - | | - | | 5 | |
| | Mass ratio | (A)/(C) | 6 | | 5 | | 3 | | 6 | | 50 | | 30 | | 5 | | 6 | | 6 | |
| | | (B)/(C) | 13 | | 11 | | 13 | | 7 | | 49 | | 69 | | 11 | | 13 | | 13 | |
| Evaluation | Viscosity (right column: mPa·s) | | A | 460 | A | 320 | B | 150 | B | 1,870 | B | 3,100 | B | 1,700 | A | 1,320 | A | 350 | B | 1,550 |
| | Compatibility (right column: minutes' seconds) | | A | 0'25 | A | 0'21 | A | 0'23 | A | 0'42 | B | 4'32 | B | 2'15 | A | 0'47 | A | 0'22 | A | 0'26 |
| | Thickness upon application (right column: total score) | | A | 56 | A | 54 | B | 43 | A | 51 | A | 53 | A | 57 | A | 58 | B | 45 | A | 56 |

**Table 2**

| | | | Comparative Examples | | | | | | | | | | | | | |
|---|---|---|---|---|---|---|---|---|---|---|---|---|---|---|---|---|
| | | | 1 | | 2 | | 3 | | 4 | | 5 | | 6 | | 7 | |
| Oil agent | Component (A) | Hydrogenated polyisobutene #1 with average carbon number of 96 | - | | 50 | | 40 | | - | | 25 | | 55 | | - | |
| | | Hydrogenated polyisobutene #2 with average carbon number of 184 | - | | - | | - | | 65 | | - | | - | | - | |
| | Component (B) | Synthesis Example 1 | 93 | | - | | 60 | | 33 | | 40 | | 44.6 | | - | |
| | Component (B') | Comparative Synthesis Example 1 | - | | - | | - | | - | | - | | - | | 100 | |
| | Component (C) | Isotetradecane | 7 | | 50 | | - | | 2 | | 35 | | 0.4 | | - | |
| | Mass ratio | *(A)*/*(C)* | 0 | | 1 | | - | | 33 | | 1 | | 138 | | - | |
| | | (B)/(C) | 13 | | 0 | | - | | 17 | | 1 | | 112 | | - | |
| Evaluation | Viscosity (right column: mPa·s) | | D | 40 | B | 2,400 | C | 5,300 | C | 10,200 | C | 80 | C | 6,100 | A | 480 |
| | Compatibility (right column: minutes' seconds) | | A | 0'15 | C | 6'20 | D | 10'42 | D | Nonuniform | A | 0'19 | C | 9'24 | C | 6'54 |
| | Thickness upon application (right column: total score) | | D | 24 | C | 37 | B | 47 | A | 58 | C | 33 | A | 55 | D | 26 |

The oil agents for cosmetic preparations of Examples 1 to 9 all had excellent viscosity, compatibility, and thickness upon application.

On the other hand, the oil agent of Comparative Example 1 had a low viscosity due to lack of component (A), which caused dripping and an insufficient thickness upon application.

The oil agent of Comparative Example 2 was insufficient in the compatibility with the silicone oil due to lack of component (B) and also insufficient in the thickness upon application.

The oil agent of Comparative Example 3 had a high viscosity due to lack of component (C), which reduced spreadability on hair or skin. Further, the oil agent of Comparative Example 3 was insufficient in the compatibility with the silicone oil.

The oil agent of Comparative Example 4 had a high viscosity due to the content of component (A) more than 60 mass%, which reduced spreadability on hair or skin. Further, the oil agent of Comparative Example 4 was insufficient in the compatibility with the silicone oil and thus could not form a uniform mixture.

The oil agent of Comparative Example 5 had a low viscosity due to the content of component (C) more than 10 mass% and the mass ratio ((B)/(C)) less than 5, which caused dripping and an insufficient thickness upon application.

The oil agent of Comparative Example 6 had a high viscosity due to the mass ratio ((B)/(C)) greater than 100, which reduced spreadability on hair or skin. Further, the oil agent of Comparative Example 6 was insufficient in the compatibility with the silicone oil.

The oil agent of Comparative Example 7 was a single-component oil agent and excellent in viscosity. However, the oil agent of Comparative Example 7 was insufficient in compatibility with the silicone oil and thickness upon application, because of not including the predetermined amounts of components (A), (B), and (C).

### [Industrial Applicability]

The oil agent for cosmetic preparations of the present invention not only has excellent compatibility with silicone oils, but also does not drip upon application, has excellent spreadability, and can provide feeling of thickness, and thus is suitable for use in hair cosmetic preparations and skin cosmetic preparations.

### (Note)

It is understood that the scope of the present invention should not be construed as being limited by the description in this specification, but should be construed only by the description of the claims. It is also understood that the contents of the patent applications and technical standards referenced in this specification are to be incorporated herein as if the contents were specifically set forth herein. Furthermore, the disclosure of Japanese Patent Application No. 2023-078941, filed on May 11, 2023, is incorporated herein by reference in its entirety.

## Claims

1. An oil agent for cosmetic preparations, comprising:
component (A): from 5 to 60 mass% of an isoparaffin having an average carbon number from 50 to 250;
component (B): from 30 to 90 mass% of an isoparaffin having an average carbon number from 20 to 24; and
component (C): from 0.3 to 10 mass% of an isoparaffin or a paraffin having 14 carbon atoms, wherein
the mass ratio of component (A) to component (C) ((A)/(C)) is from 1 to 150, and the mass ratio of component (B) to component (C) ((B)/(C)) is from 5 to 100.
